# EUROPÄISCHE PATENTSCHRIFT

(11) **EP 0 581 193 B1**
(45) Veröffentlichungstag und Bekanntmachung des Hinweises auf die Patenterteilung: **03.06.1998**
(21) Anmeldenummer: 93111732.9
(22) Anmeldetag: 22.07.1993
(51) Int. Cl.: C11D 1/94, C11D 1/90, C11D 1/74, C11D 1/29, A61K 7/08, C11D 1/83, A61K 7/50

(54) **Verfahren zur Herstellung von konzentrierten fliessfähigen und konservierungsmittelfreien Perlglanzdispersionen**
Process for the preparation of concentrated free flowing and preservative-free pearl lustre dispersions
Procédé pour la préparation des dispersions nacrées concentrées capables d'écouler, exemptes d'agents conservateurs

(30) Priorität: 27.07.1992 DE 4224715
(43) Veröffentlichungstag der Anmeldung: 02.02.1994
(73) Patentinhaber: Clariant GmbH, 65929 Frankfurt am Main (DE)
(72) Erfinder: Schmitt, Norbert, DI (FH), D-84508 Burgkirchen (DE); Reng, Alwin, DI, D-65779 Kelkheim (DE); Gohlke, Fritz Joachim, Dr., D-84508 Burgkirchen (DE)

(56) Entgegenhaltungen:
- EP-A- 0 332 805
- EP-A- 0 568 848
- WO-A-90/07323
- WO-A-92/13512
- DE-A- 3 617 306

## Beschreibung

Die Erfindung betriff ein Verfharen zur Herstellung fließfähiger konservierungsmittelfreier Perlglanzdispersionen mit Fettsäureglykolestern als Perlglanzbildner.

Es ist häufig erwünscht, Tensidkompositionen, zum Beispiel flüssigen Wasch- und Reinigungsmitteln (das sind Bodenreinigungsmittel, Geschirrspülmittel und dergleichen) oder flüssigen Körperreinigungs- und Körperpflegemitteln sowie Haarbehandlungsmitteln (das sind Shampoos, Badezusätze, Hautbehandlungslösungen und dergleichen), ein verbessertes optisches Aussehen zu verleihen. Dazu werden den Tensidkompositionen Perlglanzdispersionen (Perlglanzkonzentrate) in einer ausreichenden Menge einverleibt, wodurch sie ein perlglänzendes und damit ästhetisch ansprechendes Aussehen erhalten.

Die bekannten Perlglanzdispersionen bestehen im wesentlichen aus mindestens einer perlglanzgebenden Verbindung und mindestens einem Dispergiermittel (auch Netzmittel oder Emulgator genannt). Im Stand der Technik werden zahlreiche perlglanzbildende Verbindungen und Verbindungsmischungen empfohlen, zum Beispiel Fettsäuren, Metallsalze von Fettsäuren, Fettsäuremono- oder Fettsäuredialkanolamide, Monoester oder Diester von Ethylenglykol, Propylenglykol oder oligomeren Alkylenglykolen dieser Art mit Fettsäuren (Fettsäureglykolester), Monoester oder Polyester von Glycerin mit Carbonsäuren und Ketosulfone verschiedenster Art. Noch zahlreicher sind die Verbindungen, die als Dispergiermittel beschrieben sind, zum Beispiel Alkylsulfate, Alkylethersulfate, Alkylsulfonate, Aminoxide, Betaine, Anlagerungsprodukte von Ethylenoxid und/oder Propylenoxid an Fettalkohole, Fettsäuren oder Alkylphenole, Glycerinmono- oder Glycerindiester oder Sorbitanmono- oder Sorbitandiester von niederen oder höheren Carbonsäuren oder von Ölen, wobei die Glycerinester und Sorbitanester gegebenenfalls ethoxyliert und/oder propoxyliert sind, Alkylmono- oder Alkyloligoglykoside und dergleichen. Die bekannten fließfähigen Perlglanzdispersionen enthalten ferner zur keimtötenden (antibakteriellen, antipilzartigen und dergleichen) Ausrüstung ein oder mehrere Konservierungsmittel.

So sind in der US-Patentschrift 4,620,976 fließfähige Perlglanzdispersionen beschrieben, die eine Kombination von einem Fettsäureglykolester und einem Fettsäurealkanolamid als Perlglanzkomponente, ein System von tensidischen Verbindungen (Glykolethersulfosuccinate und Aminoxide) als Dispergiermittel und ein Konservierungsmittel zur Vermeidung einer mikrobiellen Kontamination enthalten. Trotz ihrer relativ guten Eigenschaften lassen diese Konzentrate hinsichtlich perlglanzgebender Wirkung noch zu wünschen übrig. Nachteilig ist ferner der notwendige Einsatz einer mehr oder weniger großen Menge an Konservierungsmittel.

In der kanadischen Patentschrift 2,006,248 (& WO-A-9 007 323) wird ebenfalls eine fließfähige Perlglanzdispersion beschrieben, die neben vielen möglichen Perlglanzbildnern und Dispergiermitteln 0,1 bis 5 Gew.-% von einem mehrwertigen Alkohol als kritische Komponente enthält. Auch diese Mischungen lassen hinsichtlich Perlglanz noch zu wünschen übrig und erfordern eigene Zusätze zum Schutz vor Bakterien- und/oder Pilzbefall.

In WO-A-9213512 (siehe Beispiele 4 und 7 sowie Ansprüche) werden wäßrige Perlglanzdispersionen beschrieben, die im wesentlichen a) 25 Gew.-% Ethylenglykoldistearat, b) 6 bis 7,5 Gew.-% Cocamidobetain der Formel R-CONH-(CH₂)₃-N⁺(CH₃)₂-CH₂COO⁻, c) 11 Gew.-% C₁₂₋₁₄-Alkyl-(OCH₂CH₂)₄OH und d) 20 oder 30 Gew.-% Glycerin enthalten.

Die Aufgabe der Erfindung besteht demnach darin, ein Verfahren zur Herstellung einer gut fließfähigen und damit leicht handhabbaren Perlglanzdispersion mit ausgezeichnetem Perlglanzcharakter zur Verfügung zu stellen, die keine keimtötenden Zusätze (Konservierungsmittel) erfordert, sondern schon von sich aus diesbezüglich ausgerüstet ist. Das fließfähige und konservierungsmittelfreie Perlglanzkonzentrat soll in die üblichen Tensidkompositionen ohne Schwierigkeiten inkorporiert werden können und ihnen auch schon bei Einsatz einer relativ geringen Menge an Perlglanzkonzentrat perlglänzendes Aussehen verleihen.

Es wurde überraschenderweise gefunden, daß die Kombination von ausgewählten Fettsäureglykolestern als perlglanzgebende Verbindungen und einem bestimmten Dispergiermittel mit dem ausgewählten mehrwertigen Alkohol Glycerin in einem jeweils bestimmten Anteil zur angestrebten Dispersion führt; es ist insbesondere überraschend, daß trotz einer relativ großen Menge an Glycerin zur Erreichung der keimtötenden Wirkung gute Fließbarkeit (Pumpbarkeit) und hoher Perlglanz vorliegen, was unter anderem auch aus dem speziellen Dispergiermittel resultieren dürfte, das aus einem Betain oder einem anionischen Tensid vom Sulfonat- oder Sulfat-Typ und einem Fettalkoholalkoxylat zusammengesetzt ist.

Die Perlglanzdispersion besteht im wesentlichen aus
a) 15 bis 35 Gew.-%, vorzugsweise 20 bis 30 Gew.-%, von mindestens einem Fettsäureglykolester der allgemeinen Formel 1 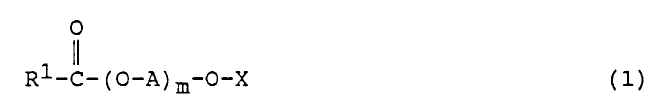 worin R¹ einen Alkylrest mit 12 bis 22 C-Atomen, A eine Gruppe der Formel -C₂H₄- oder -C₃H₆-, X ein Wasserstoffatom oder eine Gruppe der Formel 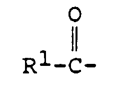 und m eine Zahl von 1 bis 10 bedeuten,
b) 5 bis 25 Gew.-%, vorzugsweise 10 bis 20 Gew.-%, von mindestens einem Betain der allgemeinen Formel 2 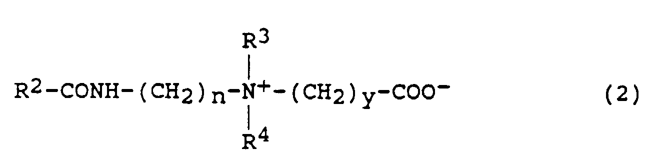 worin R² einen Alkylrest mit 8 bis 22 C-Atomen,
   R³ und R⁴ je einen Alkylrest mit 1 bis 3 C-Atomen,
   n 2, 3 oder 4 und y 1, 2 oder 3 bedeuten, oder
b') 1 bis 15 Gew.-%, vorzugsweise 2 bis 10 Gew.-%, von mindestens einem anionischen Tensid aus der Gruppe der C₆ bis C₂₂-Alkansulfonate, C₆ bis C₂₂-α-Olefinsulfonate, C₆ bis C₂₂-Fettalkoholsulfate und ethoxylierten C₆ bis C₂₂-Fettalkoholsulfate mit 1 bis 20 Ethylenoxideinheiten,
c) 5 bis 18 Gew.-%, vorzugsweise 7 bis 15 Gew.-%, von mindestens einem Fettalkoholalkoxylat der allgemeinen Formel 3

   R⁵-(O-B)_{z}-OH (3)

   worin R⁵ einen Alkylrest mit 8 bis 22 C-Atomen,
   B eine Gruppe der Formel -C₂H₄- oder -C₃H₆- und
   z eine Zahl von 1 bis 15 bedeuten,
d) 15 bis 35 Gew.-%, vorzugsweise 20 bis 30 Gew.-%, Glycerin und
e) 0 bis 50 Gew.-%, vorzugsweise 20 bis 40 Gew.-%, Wasser (Gewichtsprozente bezogen auf das Gewicht der fertigen Dispersion).

Bevorzugte Fettsäureglykolester sind solche gemäß Formel 1, wenn R¹ ein Alkylrest mit 14 bis 18 C-Atomen ist, A -C₂H₄-ist, x R¹-CO- ist und m eine Zahl von 1 bis 3 ist. Da es sich beim Alkylrest R¹ um Fettsäurereste handelt, kann dieser Rest auch Doppelbindungen enthalten, vorzugsweise 1 bis 3. Bevorzugte Vertreter der Komponente a) sind also Diester des Ethylenglykols, Diethylenglykols oder Triethylenglykols mit Palmitinsäure, Stearinsäure, Ölsäure, Talgfettsäure, Cocosfettsäure und dergleichen.

Bevorzugte Betaine sind solche gemäß Formel 2, wenn R² ein Alkylrest mit 10 bis 18 C-Atomen ist, R³ und R⁴ gleich und Methyl oder Ethyl sind, n 3 ist und y 1 ist. Da es sich beim Alkylrest R² ebenfalls um Fettsäurereste handelt, kann dieser Rest auch Doppelbindungen enthalten, vorzugsweise 1 bis 3. Bevorzugte Vertreter der Komponente b) sind also Lauroylaminopropyl-dimethylammoniumglycinat, Stearoylaminopropyl-dimethylammoniumglycinat, Cocosacylaminopropyl-dimethylammoniumglycinat, Talgacylaminopropyl-dimethylammoniumglycinat und dergleichen.

Bevorzugte Alkansulfonate, α-Olefinsulfonate und Fettalkoholsulfate sind solche mit 8 bis 18 C-Atomen in der Alkangruppe, α-Olefingruppe und Fettalkoholgruppe, wobei die ethoxylierten Fettalkoholsulfate vorzugsweise 1 bis 10 Ethylenoxideinheiten enthalten (die α-Olefinsulfonate sind bekanntlich in der Regel eine Mischung von Alken- und Hydroxyalkansulfonat). Das Kation in den Sulfonaten und Sulfaten ist vorzugsweise Natrium. Bevorzugte Vertreter der Komponente b'), die vorzugsweise aus den genannten Alkansulfonaten und/oder α-Olefinsulfonaten besteht,
sind also Natrium-C₁₃ bis C₁₇-alkansulfonat
(primäres oder sekundäres Alkansulfonat),
Natrium-C₁₄ bis C₁₆-α-olefinsulfonat und Natriumlaurylsulfat oder Natriumstearylsulfat, gegebenenfalls ethoxyliert mit 2 bis 5 mol Ethylenoxid.

Bevorzugte Fettalkoholalkoxylate sind solche gemäß Formel 3, wenn R⁵ ein Alkylrest mit 10 bis 18 C-Atomen ist, B -C₂H₄-ist und z eine Zahl von 2 bis 6 ist. Auch hier kann der Alkylrest R⁵ Doppelbindungen enthalten, vorzugsweise 1 bis 3. Bevorzugte Vertreter der Komponente c) sind also die Anlagerungsprodukte von 2 bis 6 Ethylenoxideinheiten an Decylalkohol, Laurylalkohol, Stearylalkohol,
Cocosalkylalkohol, Talgalkylalkohol und dergleichen.

Das Glycerin kann ein technisches Glycerin (das ist ein im allgemeinen bis zu 15 Gew.-% Wasser enthaltendes Glycerin) oder ein Glycerin mit höherer Reinheit sein.

Die erfindungsgemäße Herstellung der Perlglanzdispersionen erfolgt nach der folgenden Arbeitsweise. Die Komponenten b) oder b'), c) und d) und gegebenenfalls e), das sind die Betainverbindung oder das Anion-Tensid, das Fettalkoholalkoxylat, das Glycerin und das Wasser, werden in einem beheizbaren Kessel vorgelegt. Die Mischung wird unter Rühren auf 70 bis 90 °C erhitzt, um das Dispergiermittel in Lösung zu bringen. Getrennt von diesem Vorgang wird die Komponente a), das ist der Fettsäureglykolester, in einem zweiten Kessel homogen aufgeschmolzen durch Erhitzen auf eine Temperatur, die 5 bis 20 °C oberhalb des Schmelzpunktes liegt, das ist im allgemeinen eine Temperatur von 70 bis 90 °C. Die homogen aufgeschmolzene Komponente a) wird nun bei der angegebenen Temperatur von 70 bis 90 °C unter Rühren in die Mischung aus den Komponenten b) oder b'), c), d) und gegebenenfalls e) eingebracht. Die erhaltene Dispersion aus den Komponenten a) bis d) oder a) bis e) wird zur Temperierung bei einer Temperatur von 70 bis 90 °C, vorzugsweise 75 bis 85 °C, unter Rühren gehalten (etwa 20 bis 40 Minuten lang), worauf die Dispersion unter Weiterrühren auf eine Temperatur von 30 bis 40 °C abkühlen gelassen wird (dieses Abkühlen erfolgt also ohne Anwendung von Kälte). Während des Abkühlens kristallisiert die Komponente a) aus und bildet den gewünschten Perlglanz. Nach Erreichen einer Temperatur im genannten Bereich von 30 bis 40 °C wird die Dispersion unter Weiterrühren mit Hilfe von Kälte zügig (das heißt etwa 0,5 °C/min) abgekühlt bis auf Raumtemperatur (das sind etwa 20 bis 25 °C), womit die fertige, vorzugsweise Wasser enthaltende Perlglanzdispersion vorliegt.

Die vorzugsweise wäßrigen Perlglanzdispersionen können neben den beschriebenen Komponenten a) bis e) noch zweckmäßige Zusätze enthalten, zum Beispiel Puffersubstanzen und dergleichen. Sie weisen einen pH-Wert von 3 bis 8 auf.

Die Perlglanzdispersionen benötigen kein eigenes Konservierungsmittel gegen den Befall von Bakterien, Pilzen und dergleichen. Die keimtötende Eigenschaft ist bereits durch den hohen Glycerinanteil gewährleistet. Sie sind auch gut fließfähig (gießfähig, pumpfähig) und damit gut handhabbar. Aufgrund ihrer niedrigen Viskosität können die Dispersionen auf automatischen Pump-, Dosier- und Mischanlagen ohne Schwierigkeiten eingesetzt werden. Die erfindungsgemäße Dispersion besitzt einen ausgeprägten Perlglanz und ist in den eingangs erwähnten Tensidkompositionen leicht einarbeitbar, wodurch diese den angestrebten Perlglanz erhalten. Die erforderliche Menge an Perlglanzdispersion liegt bei 1 bis 10 Gew.-%, vorzugsweise 2 bis 5 Gew.-%, bezogen auf das Gewicht der zu behandelnden Tensidkomposition. Das erfindungsgemäße Perlglanzkonzentrat wird also in der angegebenen Menge den Tensidkompositionen unter Rühren zugesetzt und unter Weiterrühren darin gut verteilt.

Die Erfindung wird nun an Beispielen noch näher erläutert.

Die folgenden Beispiele 1 bis 5 geben fünf wäßrige Perlglanzdispersionen an (die Menge der einzelnen Komponenten sind Gewichtsprozente). Das Zusammenbringen der Komponenten, das heißt die Herstellung der wäßrigen Perlglanzdispersionen gemäß den Beispielen 1 bis 5, erfolgte nach der oben beschriebenen erfindungsgemäßen Arbeitsweise. Die erhaltenen wäßrigen Dispersionen wurden auf ihr Fließverhalten (Viskosität) bei 20 °C, auf ihren Perlglanz und auf ihre selbstkonservierende Eigenschaft getestet. Der Test bezüglich Fließverhalten und Perlglanz erfolgte visuell, wobei die Noten sehr gut, gut und nicht gut herangezogen wurden. Der Test auf die selbstkonservierende Eigenschaft wurde wie folgt durchgeführt:

50 g Perlglanzdispersion wurden mit 1 ml der Bakterienkultur "Pseudomonas cepacia" beimpft und anschließend homogenisiert. Die beimpften Proben wurden bei Raumtemperatur gelagert (20 °c), wobei stündlich eine Keimzahlbestimmung durchgeführt wurde (5 Stunden lang). Dazu wurde 1 g Probe mit 9 ml Caseinpepton-Lecithin-Polysorbat-Bouillon verdünnt und homogenisiert. 0,2 ml der Bouillonverdünnung wurden dann auf Caseinpepton-Sojamehlpepton-Agar aufgetragen. Nach zweitägiger Bebrütung bei 35 °c wurden die Kolonien auf den Agarplatten gezählt und die Keimzahl pro Gramm festgestellt. Die untere Nachweisgrenze des Tests liegt bei ≥ 50 kolonienbildende Einheiten pro Gramm Probe. Alle Testergebnisse sind am Ende eines jeden Beispiels angeführt.

### Beispiel 1

a) 20,0 % Monoethylenglykoldistearat
b) 15,0 % Cocamidopropylbetain
c) 14,0 % Laurylalkohol + 4 Ethylenoxid
d) 23,0 % Glycerin
e) 28,0 % Wasser
   - Fließfähigkeit:: sehr gut
   - Perlglanz:: sehr gut
   - Bakterienbefall:: nicht nachweisbar (das heißt weniger als 50 kolonienbildende Einheiten pro Gramm)

### Beispiel 2

a) 30,0 % Monoethylenglykoldistearat
b) 12,0 % Cocamidopropylbetain
c) 8,0 % Laurylalkohol + 4 Ethylenoxid
d) 27,0 % Glycerin
e) 23,0 % Wasser
   - Fließfähigkeit:: sehr gut
   - Perlglanz:: sehr gut
   - Bakterienbefall:: nicht nachweisbar

### Beispiel 3

a) 15,0 % Monoethylenglykoldistearat
b) 20,0 % Cocamidopropylbetain
c) 10,0 % Laurylalkohol + 4 Ethylenoxid
d) 20,0 % Glycerin
e) 35,0 % Wasser
   - Fließfähigkeit:: sehr gut
   - Perlglanz:: sehr gut
   - Bakterienbefall:: nicht nachweisbar

Cocamidopropylbetain ist die Kurzbezeichnung für 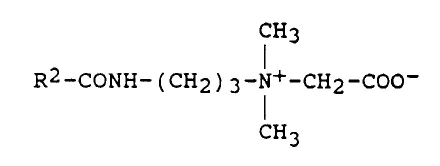
- R² =: Cocosalkyl

### Beispiel 4

a) 25,0 % Monoethylenglykoldistearat
b') 4,0 % Natrium-C₁₃ bis C₁₇-alkansulfonat
c) 10,0 % Laurylalkohol + 4 Ethylenoxid
d) 25,0 % Glycerin
e) 36,0 % Wasser
   - Fließfähigkeit:: sehr gut
   - Perlglanz:: sehr gut
   - Bakterienbefall:: nicht nachweisbar

### Beispiel 5

a) 30,0 % Monoethylenglykoldistearat
b') 6,0 % Natrium-C₁₄ bis C₁₆-α-olefinsulfonat
c) 10,0 % Laurylalkohol + 4 Ethylenoxid
d) 25,0 % Glycerin
e) 29,0 % Wasser
   - Fließfähigkeit:: sehr gut
   - Perlglanz:: sehr gut
   - Bakterienbefall:: nicht nachweisbar

## Patentansprüche

1. Verfahren zur Herstellung von konzentrierten, fließfähigen und konservierungsmittelfreien Perlglanzdispersionen bestehend im wesentlichen aus
a) 15 bis 35 Gew.-% von mindestens einem Fettsäureglykolester der allgemeinen Formel 1 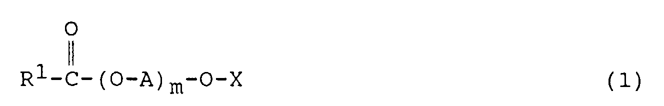 worin R¹ einen Alkylrest mit 12 bis 22 C-Atomen,
A eine Gruppe der Formel -C₂H₄- oder -C₃H₆-, X ein Wasserstoffatom oder eine Gruppe der Formel 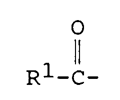 und m eine Zahl von 1 bis 10 bedeuten,
b) 5 bis 25 Gew.-% von mindestens einem Betain der allgemeinen Formel 2 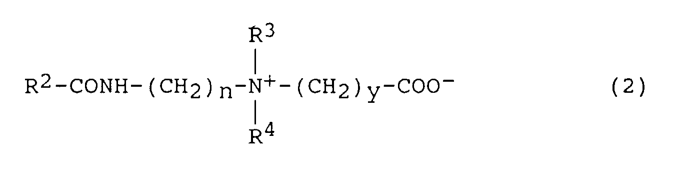 worin R² einen Alkylrest mit 8 bis 22 C-Atomen,
R³ und R⁴ je einen Alkylrest mit 1 bis 3 C-Atomen,
n 2, 3 oder 4 und y 1, 2 oder 3 bedeuten, oder
b') 1 bis 15 Gew.-% von mindestens einem anionischen Tensid aus der Gruppe der C₆ bis C₂₂-Alkansulfonate,
C₆ bis C₂₂-α-Olefinsulfonate,
C₆ bis C₂₂-Fettalkoholsulfate und ethoxylierten
C₆ bis C₂₂-Fettalkoholsulfate mit 1 bis 20 Ethylenoxideinheiten,
c) 5 bis 18 Gew.-% von mindestens einem Fettalkoholalkoxylat der allgemeinen Formel 3
R⁵-(O-B)_{z}-OH (3)
worin R⁵ einen Alkylrest mit 8 bis 22 C-Atomen,
B eine Gruppe der Formel -C₂H₄- oder -C₃H₆- und
z eine Zahl von 1 bis 15 bedeuten,
d) 15 bis 35 Gew.-% Glycerin und
e) 0 bis 50 Gew.-% Wasser,
dadurch gekennzeichnet, daß man die Komponenten b) oder b'), c) und d) und, gegebenenfalls, e) in einem beheizbaren Kessel vorlegt und die Mischung unter Rühren auf 70 bis 90 °C erhitzt, bis das Dispergiermittel gelöst ist, die Komponente a) in einem zweiten Kessel durch Erhitzen auf eine Temperatur, die etwa 5 bis 20 °C über dem Schmelzpunkt von Komponente a) liegt, verflüssigt und die flüssige Komponente a) bei 70 bis 90 °C unter Rühren in die Mischung aus den Komponenten b) oder b'), c), d) und, gegebenenfalls, e), einbringt und die erhaltene Dispersion zur Temperierung bei 70 bis 90 °C, vorzugsweise 75 bis 85 °C, etwa 20 bis 40 Minuten rührt, worauf die Dispersion zunächst unter Rühren langsam auf 30 bis 40 °C abgekühlt wird und schließlich mit einer Kühlrate von circa 0,5 °C/Minute auf 20 bis 25 °C gebracht wird, womit die fertige, vorzugsweise Wasser enthaltende Perlglanzdispersion vorliegt, die einen pH-Wert von 3 bis 8 aufweist.

## Claims

1. A process for the preparation of concentrated, flowable and preservative-free pearl luster dispersions consisting essentially of
a) 15 to 35% by weight of at least one glycol ester of a fatty acid of the formula 1 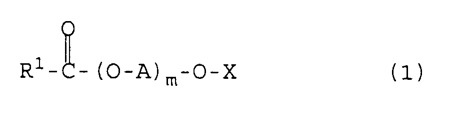 in which R¹ is an alkyl radical having 12 to 22 carbon atoms, A is a group of the formula -C₂H₄- or -C₃H₆-, X is a hydrogen atom or a group of the formula 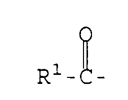 and m is a number from 1 to 10,
b) 5 to 25% by weight of at least one betaine of the formula 2 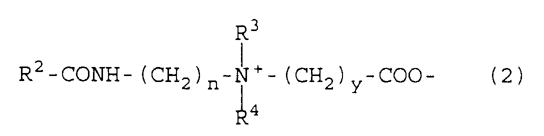 in which R² is an alkyl radical having 8 to 22 carbon atoms,
R³ and R⁴ are each an alkyl radical having 1 to 3 carbon atoms, n is 2, 3 or 4 and y is 1, 2 or 3, or
b') 1 to 15% by weight of at least one anionic surfactant from the group of the C₆- to C₂₂-alkanesulfonates, C₆- to C₂₂-α-olefinsulfonates, C₆- to C₂₂-fatty alcohol sulfates and ethoxylated C₆- to C₂₂-fatty alcohol sulfates having 1 to 20 ethylene oxide units,
c) 5 to 18% by weight of at least one fatty alcohol alkoxylate of the formula 3
R⁵-(O-B)_{z}-OH (3)
in which R⁵ is an alkyl radical having 8 to 22 carbon atoms,
B is a group of the formula -C₂H₄- or -C₃H₆- and
z is a number from 1 to 15,
d) 15 to 35% by weight of glycerol and
e) 0 to 50% by weight of water,
characterized in that components b) or b'), c) and d) and optionally e) are introduced into a heatable reactor as the initial charge and the mixture is heated to 70 to 90°C with stirring until the dispersant has dissolved, component a) is liquefied in a second reactor by heating to a temperature which is about 5 to 20°C above the melting point of component a) and the liquid component a) is introduced into the mixture of components b) or b'), c), d) and optionally e) at 70 to 90°C with stirring and the dispersion obtained is heat-treated at 70 to 90°C, preferably 75 to 85°C, for about 20 to 40 minutes with stirring, after which the dispersion is initially slowly cooled to 30 to 40°C with stirring and finally brought to 20 to 25°C at a cooling rate of approximately 0.5°C/minute, to provide the finished, preferably aqueous pearl luster dispersion, which has a pH of 3 to 8.

## Revendications

1. Procédé de préparation de dispersions nacrées fluides, concentrées et sans agent de conservation, constituées essentiellement
a) de 15 à 35 % en poids d'au moins un ester glycolique d'acide gras de formule générale 1 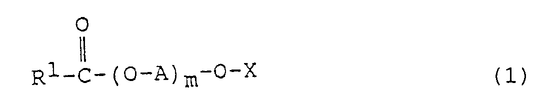 où R¹ représente un reste alkyle avec de 12 à 22 atomes de carbone, A représente un groupe de formule -C₂H₄- ou -C₃H₆-, X représente un atome d'hydrogène ou un groupe de formule 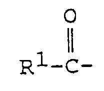 et m représente un nombre de 1 à 10,
b) de 5 à 25 % en poids d'au moins une bétaïne de formule générale 2 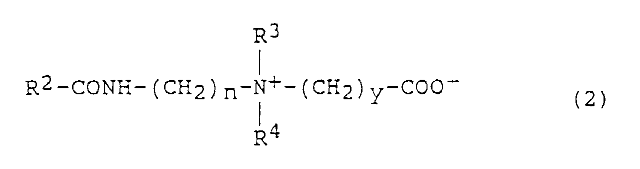 où R² représente un reste alkyle avec de 8 à 22 atomes de carbone, R³ et R⁴ représentent chacun un reste alkyle avec de 1 à 3 atomes de carbone, n vaut 2, 3 ou 4 et y vaut 1, 2 ou 3, ou
b') de 1 à 15 % en poids d'au moins un agent de surface anionique du groupe des alcanesulfonates en C₆ à C₂₂,
une α-oléfinesulfonate en C₆ à C₂₂,
des sulfates d'alcools gras ,
et des sulfates d'alcools gras éthoxylé en C₆ à C₂₂ avec de 1 à 20 unités d'oxyde d'éthylène,
c) de 5 à 18 % en poids d'au moins un alcoxylate d'alcool gras de formule générale 3
R⁵-(O-B)_{z}-OH (3)
où R⁵ représente un reste alkyle avec de 8 à 22 atomes de carbone, B représente un groupe de formule -C₂H₄- ou -C₃H₆- et z représente un nombre de 1 à 15,
d) de 15 à 35 % en poids de glycérine et
e) de 0 à 50 % en poids d'eau,
caractérisées en ce que l'on place les composants b) ou b'), c) et d), et éventuellement e), dans un récipient chauffable et on chauffe le mélange sous agitation à une température comprise entre 70 et 90 °C, jusqu'à ce que l'agent de dispersion soit dissous, on fluidifie le composant a) dans un deuxième récipient par chauffage à une température d'environ 5 à 20 °C supérieure au point de fusion du composant a), on introduit le composant a) fluide à une température de 70 à 90 °C, sous agitation, dans le mélange de composants b) ou b'), c), d), et éventuellement e), et on agite la dispersion obtenue dans un intervalle de 70 et 90 °C, de préférence de 75 et 85 °C, pour équilibrer la température, pendant environ 20 à 40 minutes, puis on refroidit la dispersion sous agitation lentement d'abord à une température de 30 à 40 °C et ensuite, à une température de 20 à 25 °C avec un taux de refroidissement d'environ 0,5 °C/minute, en obtenant la dispersion nacrée, de préférence contenant de l'eau, présentant une valeur de pH de 3 à 8.
